# EUROPÄISCHE PATENTSCHRIFT

(11) **EP 2 720 757 B1**
(45) Veröffentlichungstag und Bekanntmachung des Hinweises auf die Patenterteilung: **29.05.2019**
(21) Anmeldenummer: 12726112.1
(22) Anmeldetag: 06.06.2012
(51) Int. Cl.: A61Q 19/00, A61K 8/49, A61K 8/37

(54) **NATURKOSMETIK-KONFORME-ZUBEREITUNGEN**
COMPOSITIONS COMPLIANT TO NATURAL COSMETIC
COMPOSITIONS CONFORMES À LA COSMÉTIQUE NATURELLE

(30) Priorität: 17.06.2011 DE 102011077774
(43) Veröffentlichungstag der Anmeldung: 23.04.2014
(73) Patentinhaber: Beiersdorf AG, 20253 Hamburg (DE)
(72) Erfinder: TRILLER, Benjamin, 22941 Jersbek (DE); KERBS, Irina, 22297 Hamburg (DE)
(74) Vertreter: Hartmann, Jost
(86) Internationale Anmeldenummer: PCT/EP2012/060694
(87) Internationale Veröffentlichungsnummer: WO 2012/171838

(56) Entgegenhaltungen:
- EP-A1- 1 541 124
- EP-A2- 2 065 025
- US-A1- 2010 111 878
- DATABASE GNPD [Online] MINTEL; Mai 2009 (2009-05), "Cleansing Milk", XP002725699, Database accession no. 1112859
- DATABASE GNPD [Online] MINTEL; März 2011 (2011-03), "Meta Velour Conditioner", XP002725698, Database accession no. 1523840
- "Mackaderm (TM) GCP Multifunctional Antimicrobial Emollient Patent Pending", , 1. April 2008 (2008-04-01), XP055122074, Gefunden im Internet: URL:http://www.lipocol.com/applications/li pocolombia/archivos/98_INFORMACIN_TCNICA.p df [gefunden am 2014-06-06]
- DATABASE GNPD [Online] MINTEL; Mai 2012 (2012-05), "Firming Eye Care", XP002725688, Database accession no. 1802593
- DATABASE GNPD [Online] MINTEL; Juni 2011 (2011-06), "Make-Up Remover", XP002725697, Database accession no. 1554534
- GODFREY D ET AL: "Preservatives - Ingridients, Getting the Balance right", SOAP PERFUMERY AND COSMETICS, UNITED TRADE PRESS LTD. LONDON, GB, 1. September 2006 (2006-09-01), Seiten 51-53, XP003023642, ISSN: 0037-749X

## Beschreibung

Die naturkosmetik-konforme Zubereitung ist ohne hautschädigende Mittel formuliert und weist dennoch eine ausreichende Stabilität auf. Die kosmetische oder dermatologische Zubereitung umfasst Glycerylcaprylat und Dehydracetsäure oder deren Salz Natriumdehydroacetat und keine weiteren Konservierungsmittel.

In der Naturkosmetik werden grundsätzlich drei verschiedene Rohstoffklassen unterschieden. Zum einen sind dies natürliche Rohstoffe, die nur durch physikalische Mittel (z.B. Kaltpressen) gewonnen werden. Die zweite Klasse sind naturnahe Rohstoffe. Dies beinhaltet chemisch veränderte natürliche Rohstoffe, wobei die chemischen Reaktionen ein Vorbild in der Natur haben (z.B. Verseifung, Verersterung). Zur Sicherstellung der mikrobillen Sicherheit des Produktes können auch naturidentische d.h. vollsynthetische Konservierungsstoffe eingesetzt werden, die eine dritte Klasse an Naturkosmetikrohstoffen darstellen. Deren Gewinnung aus der Natur ist zu aufwendig. Naturidentische Rohstoffe sind synthetisch hergestellt jedoch mit dem natürlich vorkommenden Rohstoffen identisch. Hierunter fallen insbesondere organische Säuren.

Beispiele bekannter natürlicher Rohstoffe sind die in der Tabelle 1 angeführten Stoffe.

**Tabelle 1: Naturkosmetikrohstoffe**

| **Konservierungsstoff** | **Beispiele für Vorkommen in der Natur** |
|---|---|
| Ameisensäure und ihr Natriumsalz | Vorkommen in Insekten seit 1670 bekannt; wird von Käfern und anderen Gliedertieren als Wehrsubstanz verwendet. Kommt auch in Brennnesseln und Tannennadeln vor. |
| Benzoesäure, ihre Salze und ihr Ethylester | Im Benzoeharz (*Styrax benzoin*) und im Wehrsekret von Wasserkäfern (*Dytiscus sp*). |
| Benzylalkohol | Bis zu 6 % im Jasminblütenöl und sowohl frei als auch verestert in vielen anderen ätherischen Ölen. |
| Dehydracetsäure und ihre Salze | In den Blüten des Goldkelchs (*Solandra nitida, Solandra grandiflora*). |
| Propionsäure und ihre Salze | Wird bei der Propionsäuregärung gebildet. Kohlenhydrate werden durch *Lactobacillus casei, Bacillus subtilis* oder *Propionbakterium pentosaceum* zu Propionsäure umgesetzt. |
| Salicylsäure und ihre Salze | Als freie Säure in der Spierstaude (*Filipendula ulmaria*), in Sennesblättern (*Cassia angustifolia*, *Cassia senna*) und in Kamillenblüten (*Chamomilla recutita*). |
| Sorbinsäure und ihre Salze | In den Samen der Vogelbeere (Eberesche, *Sorbus aucuparia*). |

Aus der Einschränkung der Verwendung von Konservierungsmittel resultiert die Herausforderung entsprechend konservierte naturkosmetik-konforme Formeln zu entwickeln.

Wichtigste konservierende Zutaten in Kosmetika waren lange Zeit Alkohol und ätherische Öle. Allerdings sind bis zu zehn Prozent Alkohol - je nach Wasseranteil und Zutaten - nötig, um eine Lotion zu konservieren. Immer häufiger wird jedoch auf Alkohol verzichtet, weil er die Haut entfettet und austrocknet.

Konservierungsstoffe haben den Auftrag, Bakterien, Hefen und Pilze abzutöten. Entsprechend aggressiv sind sie - auch auf der Haut. Viele der künstlichen Haltbarmacher können die Haut irritieren, zu Rötungen und Ausschlag führen. Bei langjähriger Anwendung steigt die Gefahr, dass die Stoffe den Organismus sensibilisieren und es zu allergischen Reaktionen kommt.

Bekannte Allergene sind Formaldehyd und Stoffe, die im Produkt Formaldehyd freisetzen. Auch die halogenorganischen Verbindungen, zu denen viele Konservierungsmittel gehören, haben ein großes allergisches Potenzial.

Kosmetische Zubereitungen müssen langzeitstabil gegen mikrobielle Kontamination formuliert werden und trotzdem eine sehr gute Hautverträglichkeit aufweisen.

Die mikrobielle Stabilität wird bislang durch den Zusatz an Konservierungsmitteln gelöst.

Bekannte Konservierungsmittel sind die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Des Weiteren ist Benzylalkohol, Phenoxyethanol, Phenoxetol®, Ethylenglycolmonophenylether und Phenylglycol zur Konservierung von kosmetischen Mitteln bekannt.

Zubereitungen ohne diese Konservierungsmittel bereit zu stellen ist jedoch ein Wunsch der Konsumenten.

Weitere bekannte Hilfsmittel zur mikrobiellen Stabilisierung sind Benzethoniumchlorid, Lauroylethylarginat, Octopirox und Methylisothiazolinon.

Eine gesetzliche Definition des Begriffs "Naturkosmetik" gibt es weder national noch europaweit. Es gibt jedoch formelle Kriterien, welche Anforderungen an derartige Produkte zu stellen sind. Naturkosmetika sind demnach ausschließlich aus Naturstoffen herzustellen. Naturstoffe sind Substanzen pflanzlichen, tierischen und/oder mineralischen Ursprungs sowie deren Gemische und Reaktionsprodukte.

Im Internet sind die als Naturkosmetika beworbenen Produkte der Firmen Luke + Lilly Naturkosmetik (Lucky Lotion, Lovely Lotion) und NATURELLE D'ARGAN (Tagescreme) bekannt, die neben kosmetischen Bestandteilen Glyceryl Caprylat und Dehydroacetsäure umfassen. Als Konservierungsmittel umfassen diese Formulierungen jedoch immer auch Benzylalkohol.

WO 2003075883 A1 beschreibt eine kosmetische Zubereitung umfassend Retinoide und Konservierungsmittel. Die Konservierungsmittel können gewählt werden aus einer Gruppe bekannter Mittel, wie Phenole, Zinkpyrithione, Alkohole und weitere. Die Zubereitungen sind darüber hinaus parabenfrei. Neben verschiedenen anderen Stoffen und Mitteln sind Natriumdehydroacetat und Glycerylcaprylat genannt.

EP 1982689 A1 beschreibt antimikrobiell wirksame getränkte Tücher. Die Tränkungslösung liegt bei einem pH-Wert von 2,5 bis 5,4. Als Bestandteile der Tränkungslösung sind explizit u.a. Dehydroacetic acid ausgeschlossen.

Für naturkosmetik-konforme Zubereitungen gelten zwar unterschiedliche Kriterien, jedoch ist in diesen Kriterien die Zulassung von Konservierungsmitteln geregelt. Neben Benzylalkohol, der aber geruchlich nicht akzeptabel ist, sind organische Säuren wie Benzoesäure, Sorbinsäure, Dehydracetsäure oder Salicylsäure zugelassen.

Dies führt jedoch zu dem Problem, dass der Einsatz dieser Säuren im sauren Bereich unter pH 5,5 erfolgen muss. Die anti-mikrobielle Wirksamkeit von organischen Säuren ist abhängig vom pH-Wert der Formel und nur undissoziierte Säuren sind entsprechend wirksam. Bei den pKa-Werten der üblichen Säuren, wie Benzoesäure oder Sorbinsäure, ist ein pH von 5,5 als Richtwert zu sehen, bei dem eine entsprechende Wirksamkeit zu erwarten ist. Ebenso können organische Säuren ein sensuelles Missempfinden beim Konsumenten auslösen. Weiterhin ist mit Zubereitungen, enthaltend diese Säuren, ein Einsatz am Auge oder auf empfindlicher Haut nicht möglich. Die Gefahrstoffstoffeinstufung (R-Sätze) von Benzoe- oder Sorbinsäure weisen beispielsweise auf Augen- und oder Hautreizung hin. Ebenso zeigen auch interne Stinging-Test das Reizpotential dieser säurehaltigen Zubereitung.

Bei Kosmetika, die im Bereich der Augen und Schleimhäute appliziert werden, ist aber insbesondere auf deren Keimfreiheit und Verträglichkeit der Inhaltsstoffe zu achten.

Als Alternative zur Konservierung und Stabilisierung ist Alkohol in Kombination mit Konservierungsmitteln oder auch alleine in Naturkosmetikformeln zu finden. Doch auch Alkohol in anti-mikrobiell wirksamen Konzentrationen ist für die Anwendung am Auge nicht geeignet. Ethanol führt zu starken Brennen im Auge. Auch auf der Haut führt Alkohol je nach Empfindlichkeit der Haut zu Reizungen. Dies ist jedoch auch abhängig von der Lipidphase der Zubereitung. Große Lipidphasen können den Reiz-Effekt von Alkohol abfangen. Generell ist aber bei sensibler Haut kein Alkohol beim Konsumenten erwünscht.

Wünschenswert ist es daher kosmetische oder dermatologische Zubereitungen bereit zu stellen, insbesondere die zur Anwendung am Auge geeignet sind, die ohne die bekannten Konservierungsmittel stabil formuliert werden können und einen möglichst hohen pH-Wert, im Bereich des pH-Wertes der Tränenflüssigkeit (pH 7,35), aufweisen.

Die Erfindung ist eine kosmetische oder dermatologische Zubereitung umfassend Glycerylcaprylat und Dehydracetsäure und/oder deren Salz Natriumdehydroacetat. Die Zubereitung umfasst keine weiteren Konservierungsmittel.

Die Zubereitung ist frei von Parabenen, insbesondere 4-Hydroxybenzoesäure und deren Ester, Methylparaben, Ethylparaben, Propylparaben, Isopropylparaben, Butylparaben, Isobutylparaben, Phenylparaben, des Weiteren frei von Benzylalkohol, Formaldehyd, Phenoxyethanol, Ethylenglycolmonophenylether, Phenylglycol, Phenoxetol®, Benzethoniumchlorid, Lauroylethylarginat, Octopirox und/oder Methylisothiazolinon.

Der Anteil an diesen Stoffen beträgt daher weniger als 0,1 Gew.%, insbesondere weniger als 0,01 Gew.%, insbesondere 0 Gew.%, bezogen auf die Gesamtmasse der Zubereitung, um als "frei von" bezeichnet zu werden.

Ethanol ist ebenfalls ein Stoff der bevorzugt in der Zubereitung nicht enthalten ist. Da jedoch oft durch Extrakte o.ä. Rohstoffe geringe Mengen an Ethanol in die Formel eingebracht werden können, ist erfindungsgemäß eine Ethanolfreiheit mit einem Anteil von weniger als 0,1 Gew.%, bezogen auf die Gesamtmasse der Zubereitung, erreicht.

Die Zubereitung weist einen pH-Wert im Bereich 5,5 bis 7,5, bevorzugt 5,7 bis 6,5 auf.

Der pH-Wert der Zubereitung kann mit Natronlauge eingestellt werden. Durch Dehydracetsäure stellt sich ein eher saurerer pH-Wert ein. Vorteilhaft sollte kein Puffer verwendet werden bzw. die Pufferkapazität von Dehydracetsäure/ Natriumdehydroacetat so gering wie möglich gehalten werden, um Wechselwirkungen mit der Augenflüssigkeit so gering wie möglich zu halten.

Dehydroacetic acid (CAS 520-45-6, 3-acetyl-6-methylpyran-2,4(3H)-dione), Sodium Dehydroacetate (CAS 4418-26-2, Natrium-1-(3,4-dihydro-6-methyl-2,4-dioxo-2H-pyran-3-yliden)-ethanolat), ist beispielweise als Geogard ™ 111A bzw. 111S erhältlich.

### Dehydracetsäure der Struktur

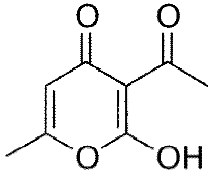

und das Natriumsalz sind als Konservierungsmittel bekannt und werden als Ersatz für Parabene verwendet.

Glyceryl Caprylat ist z.B. als Dermosoft ® GMCY erhältlich.

Glyceryl Caprylat hat hautbefeuchtende und rückfettende Eigenschaften sowie eine antimikrobielle Wirkung in einem breiten pH-Bereich. Glyceryl Caprylat kann sowohl in wässriger als auch in der Lipidphase eingesetzt werden.

Die bevorzugten Anteilsbereiche von Dehydracetsäure bzw. Natriumdehydroacetat in der kosmetischen oder dermatologischen Zubereitung liegen im Bereich von 0,3 bis 0,5 Gew.%.

Die bevorzugten Anteilsbereiche von Glyceryl Caprylate in der kosmetischen oder dermatologischen Zubereitung liegen im Bereich von 0,3 bis 0,5 Gew.%., jeweils bezogen auf die Gesamtmasse der Zubereitung.

In Stinging-, HET- und RBC- Tests sowie Hühnerei-Test an der Chorionallantoismembran (CAM) konnte gezeigt werden, dass mit Hilfe der erfindungsgemäßen Kombination von Dehydracetsäure und Glyceryl Caprylat eine sehr gute Hautverträglichkeit und ausreichende Konservierung, insbesondere im pH Bereich 5,5 - 6,5, in naturkosmetik-konformen Zubereitungen erreicht werden konnte. Dabei kann auf Zusatz von Konservierungsmitteln und Konservierungsmittelhelfer und insbesondere Alkohol gänzlich verzichtet werden.

### Stinging-Test:

Im Stinging - Test [Halbseitenvergleich Frosch u. Kligman] werden Rohstoffe und kosmetische Formulierungen auf ein mögliches sensuelles Irritationspotential untersucht und bewertet. Die Produkte werden weiblichen Probanden ("Stinger") im Wangen/Nasolabialbereich aufgetragen. Nach bestimmten Zeitintervallen teilen sie dem Versuchsdurchführenden ihre Empfindungen mit. Die Probandin ist seit mindestens 2 Stunden abgeschminkt und akklimatisiert.

Auf die linke und die rechte Gesichtshälfte wird gleichzeitig jeweils ein Produkt von der/dem Versuchsdurch führenden aufgetragen und die Probandin trägt nach vorgegebenen Zeitabständen (t = 0; 2,5; 5; 8; 15 Min.) ihr (Miss-)Empfinden in eine vorgegebene Empfindungstabelle ein.

### HET-CAM (INVITTOX Protokoll Nummer 47 und 96):

Dieser Test dient zur vergleichenden Abschätzung der Reizpotentiale von Rohstoffen und Produkten an der Chorio-Allantois-Membran (CAM) von bebrüteten Hühnereiern.

LSL-Eier (Lohmann's Selected Leghorn, Fa. Lohmann) werden am 9. Bruttag am breiten Pol geöffnet, die Eihaut entfernt und 300 µl flüssiges bzw. 100 mg festes Prüfmuster auf die CAM appliziert. Verdünnungen der Prüfmuster werden mit Kochsalzlösung oder Olivenöl hergestellt. Pro Konzentration werden 3 bzw. 4 Eier eingesetzt. Die Untersuchungen werden, abhängig vom Prüfmuster, nach dem Reaktionszeit- oder dem Feststoff-Protokoll durchgeführt. Für Prüfmuster und Verdünnungen, die transparent sind und eine kontinuierliche Beobachtung der CAM gestatten, findet das Reaktionszeit-Protokoll Anwendung (üblicherweise mit unverdünnten und 10%igen Prüfmustern). Hier werden innerhalb von fünf Minuten nach Applikation die Eintrittszeitpunkte der drei Reaktionstypen Blutung, Lysis und Koagulation, sowie ihre Schweregrade durch Beobachtung mit dem bloßen Auge bestimmt. Falls notwendig werden die Reaktionen durch Betrachtung mit einer Stereolupe (10-40 fache Vergrößerung) verifiziert. Mit einer empirisch ermittelten Formel wird aus den Reaktionszeiten der Reizindex berechnet, mit dem eine Klassifizierung des Prüfmusters zwischen "nicht reizend" bis "stark reizend" möglich ist. Zusätzlich wird die Reizschwelle bestimmt, d.h. die Konzentration, bei der die ersten schwachen Reaktionen beobachtet werden. Nichttransparente Verdünnungen bzw. Zubereitungen oder Rohstoffe werden nach dem Feststoff-Protokoll mit 5 Minuten Inkubationszeit untersucht. Nach Entfernung des Prüfmusters von der Applikationsfläche werden die Schweregrade der genannten Reaktionstypen beurteilt und das Prüfmuster entsprechend klassifiziert. Frühe Reaktionszeiten und starke Schweregrade korrelieren mit starken Reizpotentialen. Beide Protokolle sind in einer vom BGA durchgeführten Ringstudie angewendet worden (1988-1991). Abgewandelte Methoden des Hühnerei-Tests wurden in einer von der CTFA initiierten Studie bzw. in einer EC/HO-Studie geprüft.

### Red Blood Cell Test (RBC-Test):

Entsprechend dem INVITTOX Protokoll Nummer 37 ist der Red-Blood-Cell-Test EC/HO-Protokoll mit 1h Inkubation durchzuführen und dient zur Abschätzung eines möglichen in vivo Augenschleimhautreizpotentials von kosmetischen Fertigprodukten und Rohstoffen.

### 1. Rangefinding und Denaturierung

Ein definiertes Aliquot isolierter Kalbserythrozyten wird jeweils mit den Prüfmusterkonzentrationen 1, 10, 100, 1.000, 10.000, 100.000 mg/l in PBS bei pH 7.4 für eine Stunde unter Schütteln bei RT inkubiert. Nach Zentrifugation wird die Änderung der spektralen Absorption im Überstand (extrazelluläres Hämoglobin) bei 541 nm gemessen. Die Zellen im Pellet werden mit Wasser lysiert und dann die Absorption dieser Lösung (intrazelluläres Hämoglobin) ebenfalls bei 541 nm gemessen. Aus der Veränderung der Gesamtabsorption (intra- und extrazelluläres Hämoglobin) im Vergleich zum gesamten nativen Hämoglobin (HbO2) werden die Kenngrößen Dlow (die Schwellenkonzentration [mg/l] bei > 10% Denaturierung) und Dmax (die maximale Denaturierung [%]) bestimmt.

### 2. Hämolyse

Ein definiertes Aliquot isolierter Kalbserythrozyten wird jeweils mit einer Reihe steigender Konzentrationen des zu untersuchenden Prüfmusters für eine Stunde unter Schütteln bei RT inkubiert. Nach Zentrifugation werden die gewonnenen Überstände photometrisch bei 541 nm auf ihren Gehalt an freigesetztem HbO2 analysiert. Daraus wird der relative Hämolysegrad berechnet und die Konzentrations-Response-Kurve mit dem H50-Wert [mg/l] als Kenngröße bestimmt. Dieser gibt die Konzentration des Prüfmusters an, bei der 50% des Hämoglobins freigesetzt werden. Diese Methode wurde 1994/95 in zwei Validierungsstudien auf ihre Korrelation zum Kaninchenaugenschleimhaut-Reiztest (Draize-Test) untersucht. In einer Studie befanden sich Chemikalien im weitesten Sinne zur Prüfung (EC/HO-Studie), in einer weiteren Studie nur kosmetisch relevante Rohstoffe und kosmetische Fertigformulierungen (COLIPA-Studie). In der COLIPA-Studie hat der RBC-Test seine Eignung zur Vorhersage des Augenschleimhautreizpotentials von Tensiden und tensidhaltigen Formulierungen gezeigt.

Durch Dehydracetsäure bzw. Natriumdehydroacetat und Glyceryl Caprylate konnte das Problem der Reizung gelöst werden und kosmetische Zubereitungen für den Einsatz am Auge durch einen pH-Wert von über 5,5 oder auf empfindlicher Haut hergestellt werden.

Nachfolgende Untersuchungen belegen das niedrige Reizpotential erfindungsgemäßer Zubereitungen.

**Zubereitung A, RBC-Test:**

| INCI | Zubereitung A [Gew.%] |
|---|---|
| Butyrospermum Parkii Butter | 3.0000 |
| Cetearyl Alcohol | 4.2000 |
| Sodium Stearoyl Glutamate | 0.2000 |
| Caprylic/Capric Triglyceride | 3.5000 |
| Simmondsia Chinensis Seed Oil | 4.0000 |
| Prunus Amygdalus Dulcis Oil | 6.0000 |
| Glyceryl Stearate | 2.0000 |
| Xanthan Gum | 0.2000 |
| Citric Acid | 0.0450 |
| Glyceryl Caprylate | 0.3000 |
| Dehydroacetic Acid | 0.3000 |
| Aqua | 65.6300 |
| Tocopherol + Helianthus Annuus Seed Oil | 0.0250 |
| Glycerin | 9.4000 |
| Glycerin + Arctium Lappa Fruit Extract | 1.2000 |

| | |
|---|---|
| Ergebnis: Aufgrund des RBC-Test ist kein Augenschleimhautreizpotenzial zu erwarten. | |

**Zubereitung B, In-Use-Test, Stinging:**

| INCI | B [Gew.%] |
|---|---|
| Glycerin + Arctium Lappa Fruit Extract | 1.2000 |
| Cetearyl Alcohol | 4.2000 |
| Caprylic/Capric Triglyceride | 3.2000 |
| Simmondsia Chinensis Seed Oil | 4.0000 |
| Prunus Amygdalus Dulcis Oil | 5.0000 |
| Sodium Stearoyl Glutamate | 0.2000 |
| Glyceryl Stearate | 2.0000 |
| Glycerin | 9.0000 |
| Aqua + Sodium Hydroxide | 0.0200 |
| Butyrospermum Parkii Butter | 3.0000 |
| Dehydroacetic Acid | 0.3000 |
| Glyceryl Caprylate | 0.3000 |
| Xanthan Gum | 0.2000 |
| Aqua | 67.3550 |
| Tocopherol + Helianthus Annuus Seed Oil | 0.0250 |

| | |
|---|---|
| Ergebnis: Im in-use-Tests traten lediglich an 3 von 60 Probanden Unverträglichkeiten auf. | |

Die erfindungsgemäßen Zubereitungen sind besonders bevorzugt zur Anwendung am oder im Bereich um das Auge sowie auf empfindlicher Haut geeignet.

Der Anwendungsbereich um die Augen schließt einen Streifen um die Augen von etwa bis zu 3 cm ein und umfasst das Oberlid, die Nasenflügel, die Hautpartien vom Auge bis zur Schläfe und bis zur Augenbraue.

Die erfindungsgemäßen Zubereitungen sind frei von anderen Konservierungsmitteln, insbesondere Benzylalkohol-, Paraben- und Phenoxyethanolfrei und weisen dennoch eine ausreichende mikrobielle Stabilität auf.

Die kosmetischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der mikrobiologischen Stabilität sowie Haut- und Haarverträglichkeit nicht beeinträchtigt bzw. deren Anteil zu vermeiden ist.

Die erfindungsgemäßen Zubereitungen umfassen vorzugsweise nur Naturstoffe, natürliche, naturnahe und/oder naturidentische Rohstoffe. Naturstoffe sind Substanzen pflanzlichen, tierischen und/oder mineralischen Ursprungs sowie deren Gemische und Reaktionsprodukte. Als natürliche, naturnahe und/oder naturidentische Rohstoffe sind Stoffe anzusehen, die aus Naturstoffen nur durch physikalische Mittel gewonnen werden, nur chemisch verändert werden bzw. naturidentisch hergestellt werden.

Erfindungsgemäße kosmetische und dermatologische Zubereitungen können in verschiedenen Formen vorliegen. So können sie z. B. eine Lösung, eine Emulsion oder Mikroemulsion vom Typ Wasser-in-ÖI (W/O), eine multiple Emulsion, beispielsweise vom Typ Wasser-in-ÖI-in-Wasser (W/O/W), eine Emulsion vom Typ Öl-in-Wasser (O/W) oder eine Gelemulsion bzw. Hydrodispersionsgel darstellen. Erfindungsgemäß besonders vorteilhaft sind kosmetische und dermatologische Zubereitungen in Form von Öl-in-Wasser (O/W).

Nachfolgende Beispiele veranschaulichen die erfindungsgemäßen Zubereitungen. Die Angaben sind Gewichtsanteile jeweils bezogen auf die Gesamtmasse der Zubereitung.

### Beispiele

| INCI | C [Gew.%] |
|---|---|
| Cetearyl Alcohol | 3.0000 |
| Caprylic/Capric Triglyceride | 5.0000 |
| Octyldodecanol | 5.0000 |
| Sodium Stearoyl Glutamate | 0.2000 |
| Glyceryl Stearate | 3.0000 |
| Glycerin | 8.0000 |
| Aqua + Sodium Hydroxide | 0.0250 |
| Butyrospermum Parkii Butter | 2.0000 |
| Dehydroacetic Acid | 0.3000 |
| Glyceryl Caprylate | 0.5000 |
| Xanthan Gum | 0.3000 |
| Aqua | 72.6750 |

| INCI | D [Gew.%] |
|---|---|
| Cetearyl Alcohol | 3.0000 |
| Caprylic/Capric Triglyceride | 4.0000 |
| Octyldodecanol | 4.0000 |
| Simmondsia Chinensis Seed Oil | 2.0000 |
| Potassium Cetyl Phosphate | 0.2000 |
| Glyceryl Stearate | 3.0000 |
| Glycerin | 8.0000 |
| Aqua + Sodium Hydroxide | 0.1050 |
| Butyrospermum Parkii Butter | 2.0000 |
| Dehydroacetic Acid | 0.5000 |
| Glyceryl Caprylate | 0.3000 |
| Xanthan Gum | 0.2000 |
| Aqua | 70.7600 |

| INCI | E [Gew.%] |
|---|---|
| Cetearyl Alcohol | 4.0000 |
| Prunus Amygdalus Dulcis Oil | 12.0000 |
| Potassium Cetyl Phosphate | 0.3000 |
| Glyceryl Stearate | 3.0000 |
| Glycerin | 10.0000 |
| Aqua + Sodium Hydroxide | 0.1050 |
| Butyrospermum Parkii Butter | 2.0000 |
| Dehydroacetic Acid | 0.3000 |
| Glyceryl Caprylate | 0.3000 |
| Xanthan Gum | 0.2000 |
| Tocopherol | 0.0500 |
| Aqua | 67.5450 |

## Patentansprüche

1. Kosmetische oder dermatologische Zubereitung umfassend Glycerylcaprylat und Dehydracetsäure und/oder Natriumdehydroacetat **dadurch gekennzeichnet, dass** die Zubereitung frei ist von Parabenen, Benzylalkohol, Formaldehyd, Phenoxyethanol, Ethylenglycolmonophenylether, Phenylglycol, Benzethoniumchlorid, Lauroylethylarginat, Octopirox und Methylisothiazolinon, weniger als 0,1 Gew.% Ethanol umfasst und einen pH-Wert im Bereich von 5,5 bis 7,5 aufweist.

2. Zubereitung nach einem der vorstehenden Ansprüche, **dadurch gekennzeichnet, dass** der Anteil an Dehydracetsäure und Natriumdehydroacetat im Bereich von 0,3 bis 0,5 Gew.% und an Glycerylcaprylat im Bereich von 0,3 bis 0,5 Gew.%, jeweils bezogen auf die Gesamtmasse der Zubereitung, gewählt werden.

3. Zubereitung nach einem der vorstehenden Ansprüche, mit einem pH Wert im Bereich von 5,7 bis 6,5.

4. Zubereitung nach einem der vorstehenden Ansprüche, umfassend neben Glycerylcaprylat und Dehydracetsäure und/oder Natriumdehydroacetat nur Naturstoffe, natürliche, naturnahe und/oder naturidentische Rohstoffe.

5. Nicht-therapeutische kosmetische Verwendung der Zubereitung nach einem der Ansprüche 1 bis 4 im Bereich um die Augen.

## Claims

1. Cosmetic or dermatological preparation comprising glyceryl caprylate and dehydroacetic acid and/or sodium dehydroacetate, **characterized in that** the preparation is free of parabens, benzyl alcohol, formaldehyde, phenoxyethanol, ethylene glycol monophenyl ether, phenyl glycol, benzethonium chloride, ethyl lauroyl arginate, octopirox and methylisothiazolinone, comprises less than 0.1% by weight ethanol and has a pH in the range of 5.5 to 7.5.

2. Preparation according to any of the preceding claims, **characterized in that** the fraction of dehydroacetic acid and sodium dehydroacetate is selected in the range of 0.3 to 0.5% by weight and that of glyceryl caprylate is selected in the range of 0.3 to 0.5% by weight, based in each case on the total mass of the preparation.

3. Preparation according to either of the preceding claims, having a pH in the range of 5.7 to 6.5.

4. Preparation according to any of the preceding claims comprising, in addition to glyceryl caprylate and dehydroacetic acid and/or sodium dehydroacetate, only natural substances, natural, near-natural and/or nature-identical raw materials.

5. Non-therapeutic cosmetic use of the preparation according to any of Claims 1 to'4 in the area around the eyes.

## Revendications

1. Préparation cosmétique ou dermatologique comprenant du caprylate de glycéryle et de l'acide déshydracétique et/ou du déshydroacétate de sodium, **caractérisée en ce que** la préparation est exempte de parabènes, d'alcool benzylique, de formaldéhyde, de phénoxyéthanol, d'éther monophénylique d'éthylène glycol, de phénylglycol, de chlorure de benzéthonium, d'arginate de lauroyléthyle, d'octopirox et de méthylisothiazolinone, comprend moins de 0,1 % en poids d'éthanol et présente un pH dans la plage allant de 5,5 à 7,5.

2. Préparation selon l'une quelconque des revendications précédentes, **caractérisée en ce que** la proportion d'acide déshydracétique et de déshydroacétate de sodium est choisie dans la plage allant de 0,3 à 0,5 % en poids et la proportion de caprylate de glycéryle dans la plage allant de 0,3 à 0,5 % en poids, à chaque fois par rapport à la masse totale de la préparation.

3. Préparation selon l'une quelconque des revendications précédentes, ayant un pH dans la plage allant de 5,7 à 6,5.

4. Préparation selon l'une quelconque des revendications précédentes, comprenant, outre le caprylate de glycéryle et l'acide déshydracétique et/ou le déshydroacétate de sodium, uniquement des substances naturelles, des matières premières naturelles, proches du naturel et/ou identiques au naturel.

5. Utilisation cosmétique non thérapeutique de la préparation selon l'une quelconque des revendications 1 à 4 dans la zone du contour des yeux.
